# EUROPEAN PATENT APPLICATION

(11) **EP 4 663 290 A1**
(43) Date of publication of application: **17.12.2025**
(21) Application number: 24752947.2
(22) Date of filing: 31.01.2024
(51) Int. Cl.: B01J 23/46, B01J 37/02, B01J 37/04, B01J 37/08, C07C 1/12, C07C 9/04

(54) **CATALYST CONTAINING RUTHENIUM-OXO-CARBONATE AS AN ACTIVE PHASE FOR THE HYDROGENATION OF CO2 TO FORM METHANE AT LOW REACTION TEMPERATURES**

(30) Priority: 10.02.2023 ES 202330102
(71) Applicant: Consejo Superior De Investigaciones Científicas (CSIC), 28006 Madrid (ES); Universitat Politècnica de València, 46022 Valencia (ES)
(72) Inventor: CORMA CANÓS, Avelino, 46022 Valencia (ES); HEYDORN, Patricia Concepción, 46022 Valencia (ES); TÉBAR SOLER, Carmen, 46022 Valencia (ES)
(74) Representative: Pons IP
(86) International application number: PCT/ES2024/070053
(87) International publication number: WO 2024/165773

(57) **Abstract**

The invention relates to a catalyst containing ruthenium-oxo-carbonate, the production method thereof and its use for the hydrogenation of CO₂ to form methane at temperatures < 200°C.

## Description

### Field of the invention

The present invention relates to a transition metal oxide-based catalyst, for the hydrogenation of CO₂ to form methane at low temperatures (< 200°C). More particularly, it relates to a monoclinic ruthenium oxide catalyst containing carbon in its crystal structure. This phase is considered active for the activation of CO₂ at low temperatures, selectively giving methane as a hydrogenation product.

### Background of the invention

The use of CO₂ has gained much attention as an alternative carbon source to the use of fossil fuels for the synthesis of chemicals and fuels. Among the different catalytic processes, obtaining methane by hydrogenation of CO₂ is of particular interest from the point of view of storing surplus renewable energy in the form of CH₄, this surplus being easily transportable in current driving systems. Most of the processes for obtaining methane by hydrogenation of CO₂ are thermal processes that, unlike electro-catalytic and photo-catalytic processes, are much more efficient. The catalysts proposed in the literature for producing CH₄ from CO₂ are based on metals such as Ni, Ru, Pd, Rh, mono- or multimetallic metals, metals with or without promoters (Na, K, Cs, rare earth elements, etc.) supported on different metal oxides (TiO₂, SiO₂, Al₂O₃, CeO₂, ZrO₂) or carbon materials (N-doped CNT) (CN108686635A; CN109433199A; CN108927194A; US3884838A). These catalysts require high reaction temperatures (250 -450°C), low space velocity (600 h⁻¹) and usually operate in excess hydrogen (H₂/CO₂= 11-5) to increase the methane yield. For example, patent document CN1107078A describes a catalyst composed of Ni⁰ (2 - 40%), Ru⁰ (0.1 - 7 wt%) and sepiolite (60 - 98%), pre-activated in H₂ at 400°C for 1 hour, operating at reaction temperatures of 400 - 450°C, space velocity of 6000 - 9000 h⁻¹, atmospheric pressure and H₂/CO₂ molar ratio of 4, giving a space-time yield of methane of 58 mol·L⁻¹·h⁻¹. Patent document JP01261202 describes a ruthenium catalyst in metallic phase on an alumina supporter, which operates at 300 - 400°C, low space velocity (670 h⁻¹) and pressure of 0.2 MPa, resulting in 100% conversion of CO₂. In most of these cases, the catalysts are pre-activated in H₂ stream at temperatures of (250 - 450°C) to generate the corresponding metal species which act as active centres. In addition to catalysts based on metal particles as active centres, other studies refer to metal carbides as active centres in the activation of CO₂ (Catal. Lett. 2015, 145, 1365-1373). For example, Corma et al. (JACS 2019, 141, 49, 19304-19311) describe a catalyst containing a ruthenium metal core with a ruthenium carbide layer on the outside. The catalyst operates at reaction temperatures of 160°C, atmospheric pressure, 21428 h⁻¹ GHSV and H₂/CO₂ ratio of 3, giving a space-time yield of methane of 8.8 mol_{CH4}·L⁻¹·h⁻¹ (0.01 mol_{CH4}·h⁻¹g_{cat}⁻¹) (Patent document ES2828458). In other studies, the use of transition metal oxides as catalysts for the hydrogenation of CO₂ to form methane is described. For example, H.K. Pawlak et al. (J. of CO2 utilization 2017, 17, 312-319) describe the synthesis of thin films of nano-particulate metal oxides between 4-8 nm of RuO₂, Co₃O₄, Fe₃O₄ and mixed oxides RuO₂/Co₃O₄, for producing methane from CO₂/H₂. In this study, the conversion of CO₂ takes place at reaction temperatures above 200°C, reporting values of 40% conversion of CO₂ at 350°C, GHSV = 3000 cm³·h⁻¹·g_{cat}⁻¹ and H₂/CO₂ molar ratio of 4 in the case of RuO₂. J. Polanski et al. (Appl. Catal. B. Environ., 2017, 206, 16-23) report 100% conversion of CO₂ with 100% selectivity to methane and a space yield of STY_{CH4} 0.123 mol_{CH4}·h⁻¹·g_{cat}⁻¹ at 200°C, H₂/CO₂ = 4 and atmospheric pressure, on catalysts of 1.5 wt% ruthenium oxide (RuO₂) supported on nickel oxide (NiO). However, these catalysts are not stable under reaction conditions and deactivate with the reaction time. Patent document US4847231A1 claims a catalyst composed of Ru/RuO_{X}, (0>X>2), supported on different metal oxides (TiO₂, ZrO₂, HfO₂, BaTiO₃, Al₂O₃) for the hydrogenation reaction of CO₂ to form methane in gas phase, in the presence or absence of light, operating at low temperatures and low pressure, with 100% selectivity to methane. In the case of TiO₂/Ru/RuOₓ, methane production is 1.7*10⁻⁶ mol_{CH4}·h⁻¹·g_{cat}⁻¹ at 25°C and 0.1*10⁻³ mol_{CH4}·h⁻¹ g_{cat}⁻¹ at 90°C, 1 bar and in the absence of light. Other studies report reaction temperatures below 200°C for the hydrogenation of CO₂ to form methane, however, the obtained methane yields are low. Therefore, D.P. Becker et al. (Catal. Sci.Technol. 2016, 6, 8117-8128) describe a Ru/TiO₂ catalyst synthesised by impregnating a colloidal suspension of RuO₂ nanoparticles in TiO₂ (P25) with yields of 0.003 mol_{CH4}·h⁻¹·g_{cat}⁻¹ at 165°C and 0.009 mol_{CH4}·h⁻¹·g_{cat}⁻¹ at 200°C operating at a contact time of 1.6 ml·s⁻¹·g⁻¹ (F/W), H₂/CO₂ = 4 and atmospheric pressure. Patent document CN111514889A describes supported ruthenium catalysts using ionic liquids in their synthesis allowing high dispersion of the ruthenium active centres. The authors report high stability and activity at low reaction temperatures. For example, in the case of Ru catalysts using [EMIM]CI as an ionic liquid and supported on SiO₂, 3.7% conversions of CO₂ are obtained at 180°C, space velocity of 2400 h⁻¹, H₂:CO₂:N₂ molar ratio = 4:1:5 and 1 bar, after pre-activation of the catalyst at 400°C in H₂ for 3 hours.

In summary, the yields reported so far operating at low reaction temperatures, below 200°C, are low, being below 0.02 mol_{CH4}·h⁻¹·g_{cat}⁻¹.

The present invention relates to a novel material containing at least Ru metal, an inorganic matrix and a new crystalline phase defined as Ru oxo-carbonate, the latter acting as an active phase in the activation of CO₂. This material is active and selective in the hydrogenation of CO₂ to form methane at low temperatures (< 200°C).

The fields of application include the composition of the active phase of the catalyst, the composition of the catalyst, the methods for obtaining the catalyst and the application thereof to obtain methane from CO₂ by thermal hydrogenation.

The present invention, compared to the state of the art, has the following advantages: 1) good activity and selectivity to methane at low temperature (< 200°C); 2) high stability over time; 3) the method of preparation does not involve a pre-activation step of the pre-catalyst at high temperatures.

### Description of the invention

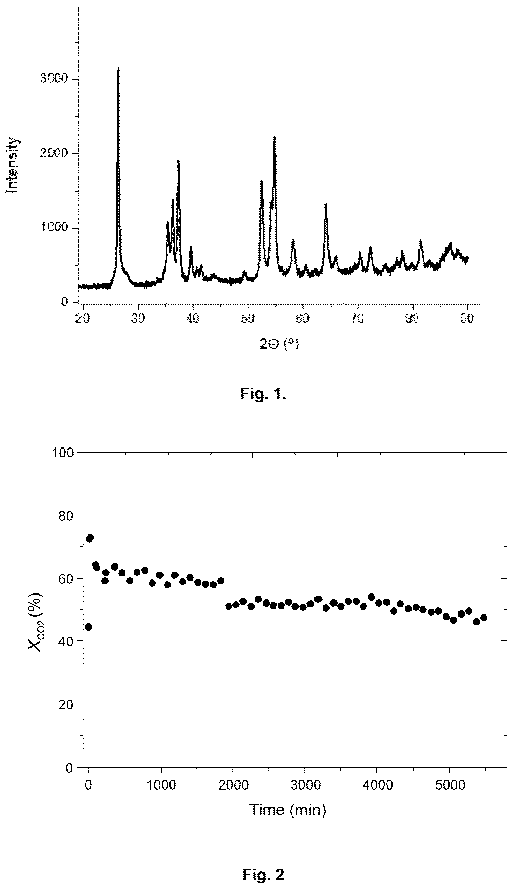

The present invention describes a catalyst containing a new crystalline phase of ruthenium oxide, which crystallises in a monoclinic structure containing carbon in interstitial positions. The crystal structure determined by X-ray diffraction is shown in Figure 1 of said invention. It is monoclinic ruthenium oxide, with space group P2₁/c. The cell parameters, fractional atomic coordinates and bond lengths are included in tables 1-3. Rietveld refinement of the X-ray pattern results in a RuO₂C_{X} composition, where X is between 0.3-0.8; preferably 0.4-0.8. The presence of carbon in interstitial positions defines the formation of a ruthenium oxo-carbonate.

A first aspect of the present invention therefore relates to a ruthenium catalyst comprising, at least:
- Ru metal
- An organic or inorganic matrix which is preferably selected from silica, alumina, metal oxides, zeolites, carbon and combinations thereof, and more preferably, it is carbon.
- Ru oxo-carbonate which acts as an active phase and has the following structure:

   RuOₓC_{y}

Where x is between 1 and 3, preferably between 1.8 and 2.5.
Where y is between 0.3 and 0.8, preferably between 0.4 and 0.8.

According to a particular embodiment, the active phase of the catalyst described may further comprise nitrogen in an amount between 0.1-0.5 molar ratio, preferably between 0.2 and 0.4.

This active phase crystallises in a monoclinic phase containing carbon in interstitial positions.

According to a preferred embodiment, the catalyst described above may further comprise a metal preferably selected from Pd, Co, Ni, In, alkalis, alkaline earth metals and combinations thereof, between 0.2 and 98 wt% of the total metal component.

A second aspect of the present invention relates to the method for obtaining the catalyst described above.

Said catalyst can be obtained directly by a process comprising, at least, bringing a ruthenium pre-catalyst preferably selected from: RuO₂, a mixture of RuO₂ with an organic or inorganic matrix and a mixture of RuO₂ with other metal oxides selected from PdO₂, Co₃O₄, NiO, In₂O₃, alkalis, alkaline earth metals and combinations thereof, into contact with a reaction gas mixture comprising, at least, H₂, N₂ and CO₂.

As described, the composition of the pre-catalyst according to the present invention may comprise a single phase, for example, ruthenium oxide, or two phases, that is, the pre-catalyst may be selected from RuO₂, a mixture of RuO₂ with an organic or inorganic matrix and a mixture of RuO₂ with other metal oxides selected from PdO₂, Co₃O₄, NiO, In₂O₃, alkalis, alkaline earth metals and combinations thereof.

According to a preferred embodiment, the pre-catalyst is a mixture of RuO₂ with an organic or inorganic matrix in a proportion by weight between 99.9 and 10 RuO₂: and 0.1 and 90 organic or inorganic matrix, more preferably between 80 and 50 RuO₂: and 20 and 50 organic or inorganic matrix.

This pre-catalyst can be a physical mixture between RuO₂ and the organic or inorganic matrix, mixed by physical methods, such as ball mills, or chemical methods, such as impregnation, co-precipitation, sol-gel.

According to a particular embodiment, the RuO₂ / organic or inorganic matrix ratio is between 10:90, and preferably between 75 and 25.

According to a particular embodiment, the H₂/CO₂ molar ratio in the reaction gas mixture may be between 1 and 6, preferably between 2 and 4, and more preferably, it is 3.

According to a particular embodiment of the method for obtaining the catalyst, the pre-catalyst is brought directly into contact with the reaction gas mixture without first undergoing any pre-activation step.

According to another particular embodiment, the pre-catalyst can be subjected to a hydrothermal method, before being brought into contact with the reaction gas mixture, which can be carried out in an autoclave, at a temperature between 150-250°C, preferably between 175-200°C under static conditions for 10-30 hours, preferably between 18 and 24 hours. Furthermore, the hydrothermal method to which the pre-catalyst is subjected may comprise an organic compound which may be selected from a monosaccharide selected from glucose, mannose, fructose, xylose, a sugar oligomer, an organic compound containing at least two nitrogen atoms (e.g., ethylenediaminetetraacetic acid, chitosan, alginate, urea or combinations, among others) and combinations thereof, and a polar solvent which may be selected from water or mixtures of water with short-chain alcohols in a ratio between 1:1 to 5: 1 (v/v), preferably 1:1.

The catalyst object of the invention is active and selective in the hydrogenation of CO₂ to form methane at reaction temperatures between 50 ° - 200°C, preferably between 120° - 200°C, and more preferably between 150° and 190°C. Thus, a third aspect of the invention relates to the use of the catalyst described above for obtaining methane by hydrogenation of CO₂ at a temperature between 120 and 200°C, preferably between 150 and 190°C, and a space velocity (GHSV) between 10714 and 42857 h⁻¹, preferably between 12857 - 32142 h⁻¹, in the amount of hydrogen (H₂/CO₂ < 6, preferably 3) and a pressure between atmospheric and 5 MPa, which can be carried out in a fixed bed reactor, fluidised bed, slurry reactor or in a membrane reactor.

According to a particular embodiment, the hydrogenation reaction of CO₂ can take place in a fixed-bed flow reactor, with a catalyst particle size of 400 to 600 µm, diluted in SiC (screened between 600 and 800 µm) in a proportion by weight (catalyst: SiC) = 0.04-0.25, more preferably 0.14.

The space-time yield (STY) of methane in the present invention (see example 1) operating at 2 MPa is, in the case of the physical mixture RuO₂+C, 117.04 mol_{CH4}·L⁻¹·h⁻¹ (31.21 mol·h⁻¹·g_{cat}⁻¹) at 180°C operating at H₂/CO₂ = 3 and 24000 h⁻¹ h⁻¹. These values are notably higher than those obtained in other conventional catalysts for obtaining methane by hydrogenation of CO₂. The selectivity to CH₄ is 99.9% in all cases.

The stability of the catalyst under reaction conditions can be modified by the addition of an organic or inorganic matrix or additives and/or reaction conditions (see examples 1, 2 and 3).

### Tables 1-3:

**Table 1: Unit cell data of the ruthenium oxo-carbonate structure.**

| |
|---|
| C_{0.809}O₄Ru₂ |
| Monoclinic, *P*2₁/*c* |
| a = 5.06776 (12) Å |
| *b* = 5.66760 (11) Å |
| c = 9.09652 (19) Å |
| b = 89.928 (8) ° |
| *V* = 261.27 (1) Å³ |
| Z=4 |
| *R*ₚ = 7.1% |
| *R*_{wp} = 9.1% |

**Table 2: Fractional atomic coordinates and isotropic or equivalent isotropic displacement parameter.**

| | *x* | *y* | *z* | *U*ᵢₛₒ*/*U*_{eq} | Occupancy |
|---|---|---|---|---|---|
| Ru1 | 0.5014 (18) | 0.7828 (2) | -0.0007 (15) | 0.0100* | 1 |
| Ru2 | 0.0043 (11) | 0.9954 (10) | 0.74662 (16) | 0.0100* | 1 |
| O1 | 0.145 (10) | 0.753 (7) | 0.888 (6) | 0.0225* | 1 |
| O2 | 0.366 (5) | 0.497 (14) | 0.658 (2) | 0.0225* | 1 |
| O3 | 0.367 (5) | 0.500 (18) | 0.1263 (18) | 0.0225* | 1 |
| O4 | 0.868 (12) | 0.740 (7) | 0.608 (7) | 0.0225* | 1 |
| C7 | 0.69300 | 0.51170 | 0.11570 | 0.0250* | 0.44 (3) |
| C8 | 0.21400 | 0.50430 | 0.47670 | 0.0250* | 0.37 (2) |

**Table 3. Bond length data (Å).**

| | | | |
|---|---|---|---|
| Ru1-Ru1¹ | 2.462 (2) | O2-Ru1^{vii} | 2.02 (5) |
| Ru1-O1ⁱⁱ | 2.08 (6) | O2-Ru2^{xii} | 2.07 (3) |
| Ru1-O2ⁱⁱⁱ | 1.99 (5) | O2-C8 | 1.821 (19) |
| Ru1-O2^{iv} | 2.02 (5) | O3-Ru1 | 2.09 (8) |
| Ru1-O3 | 2.09 (8) | O3-Ru1^{v} | 2.08 (8) |
| Ru1-O3^{v} | 2.08 (8) | O3-Ru2^{iv} | 2.14 (2) |
| Ru1-O4^{iv} | 2.11 (7) | O3-C7 | 1.66 (3) |
| Ru1-C7 | 2.104 (8) | O4-Ru1^{vii} | 2.11 (7) |
| Ru1-C7^{v} | 2.203 (7) | O4-Ru2^{xv} | 2.04 (6) |
| Ru1-C8ⁱⁱⁱ | 1.925 (7) | O4-Ru2^{xvi} | 2.02 (5) |
| Ru1-C8^{iv} | 1.903 (7) | O4-C7^{vii} | 1.66 (4) |
| Ru2-O1 | 2.01 (5) | O4-C8^{xiii} | 1.64 (5) |
| Ru2-O1^{vi} | 2.05 (5) | C7-Ru1 | 2.104 (8) |
| Ru2-O2^{vi} | 2.07 (3) | C7-Ru1^{v} | 2.203 (7) |
| Ru2-O3^{vii} | 2.14 (2) | C7-Ru2^{xvii} | 1.978 (4) |
| Ru2-O4^{viii} | 2.04 (6) | C7-O1^{xiii} | 1.71 (4) |
| Ru2-O4^{ix} | 2.02 (5) | C7-O3 | 1.66 (3) |
| Ru2-C7^{x} | 1.978 (4) | C7-O4^{iv} | 1.66 (4) |
| O1-Rul^{xi} | 2.08 (6) | C8-Ru1^{xiv} | 1.925 (7) |
| O1-Ru2 | 2.01 (5) | C8-Ru1^{vii} | 1.903 (7) |
| O1-Ru2^{xii} | 2.05 (5) | C8-O1^{iv} | 1.63 (5) |
| O1-C7^{xiii} | 1.71 (4) | C8-O2 | 1.821 (19) |
| O1-C8^{vii} | 1.63 (5) | C8-O4^{xiii} | 1.64 (5) |
| O2-Ru1^{xiv} | 1.99 (5) | | |

### Brief description of the figures

Figure 1. X-ray diffraction of the ruthenium oxo-carbonate phase.
Figure 2. Conversion of CO₂ from the pre-catalyst A brought into contact with a reaction gas mixture at 180°C, 2 MPa, 24000 h⁻¹ space velocity and the reaction gas composition of 23.8 vol% CO₂, 71.3 vol% H₂, 5 vol% N₂.
Figure 3. X-ray diffraction of the pre-catalyst A and after being subjected to the reaction gas mixture at 2 MPa, 180°C and 24000 h⁻¹ space velocity.
Figure 4. Conversion of CO₂ from the pre-catalyst A' brought into contact with a reaction gas mixture at 180°C, 2 MPa, 24000 h⁻¹ space velocity and the reaction gas composition of 23.8 vol% CO₂, 71.3 vol% H₂, 5 vol% N₂.
Figure 5. Conversion of CO₂ from the pre-catalyst A brought into contact with a reaction gas mixture at 180°C, 0.1 MPa, 24000 h⁻¹ space velocity and the reaction gas composition of 23.8 vol% CO₂, 71.3 vol% H₂, 5 vol% N₂.
Figure 6. Conversion of CO₂ from the pre-catalyst B brought into contact with a reaction gas mixture at 180°C, 2 MPa, 24000 h⁻¹ space velocity and the reaction gas composition of 23.8 vol% CO₂, 71.3 vol% H₂, 5 vol% N₂.
Figure 7. Conversion of CO₂ from the pre-catalyst C brought into contact with a reaction gas mixture at 180°C, 2 MPa, 24000 h⁻¹ space velocity and the reaction gas composition of 23.8 vol% CO₂, 71.3 vol% H₂, 5 vol% N₂.
Figure 8. Conversion of CO₂ to reference catalyst D at 180°C, 2 MPa, 24000 h⁻¹ space velocity and the feed composition of 23.8 vol% CO₂, 71.3 vol% H₂, 5 vol% N₂.
Figure 9. X-ray diffraction of the reference catalyst D after being subjected to the reaction gas mixture at 2 MPa, 180°C and 24000 h⁻¹ space velocity.

### DESCRIPTION OF EMBODIMENTS

### EXAMPLE 1. Pre-catalyst A

The pre-catalyst A is a physical mixture of RuO₂ from Aldrich (99.9%) with charcoal (Norit), in the proportion of 75:25 by weight. The mixture is made by mixing both components in a mortar for 30 min. The particle size of RuO₂ according to the X-ray diffractogram is ~38 nm.

### EXAMPLE 2. Catalytic behaviour of the pre-catalyst A in contact with the reaction gases at 2 MPa (Figure 2)

The catalytic behaviour of the catalyst is tested in a stainless steel fixed-bed reactor with an inner diameter of 11 mm and a length of 240 mm. 260 mg (400-600 µm particle size) of pre-catalyst are diluted with 6300 mg SiC (600-800 µm particle size). The volume occupied by the pre-catalyst is 0.28 cm³. No prior activation is required. The resulting mixture is brought into contact with 23.8 ml min⁻¹ CO₂, 71.3 ml min⁻¹ H₂ and 5 ml min⁻¹ N₂. The space velocity is 24000 h⁻¹, temperature is 180°C and pressure is 2 MPa. Multiple analyses, each with a duration of 15 minutes, are acquired for a total of 5475 min of reaction time. Figure 2 shows the time evolution in the conversion of CO₂ at 180°C, 2 MPa and 24000 h⁻¹. Selectivity to methane is 99.9%.

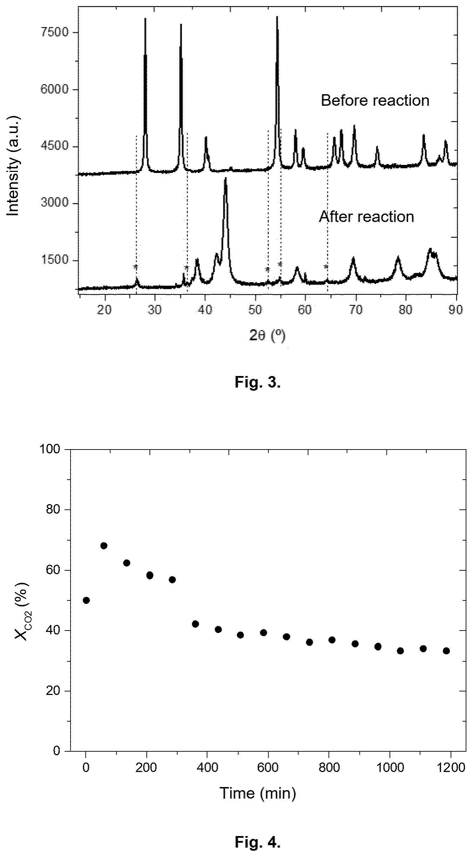

The data in **Figure 2** corresponds to ~50% conversion of CO₂ under steady-state conditions, and a methane yield of 117.04 mol_{CH4}·L⁻¹·h⁻¹ (31.21 µmol·s⁻¹·g_{cat}⁻¹). The X-ray diffraction of the catalyst after subjecting the pre-catalyst to the reaction gas mixture is shown in **Figure 3****.** The peaks associated with the ruthenium oxo-carbonate phase are asterisked.

### EXAMPLE 3. Pre-catalyst A'

The pre-catalyst A' is a physical mixture of RuO₂ from Aldrich (99.9%) with SiO₂ (Aldrich, 99.5%)., in the proportion of 75:25 by weight. The mixture is made by mixing both components in a mortar for 30 min. The particle size of RuO₂ according to the X-ray diffractogram is ~38 nm. SiO₂ has a particle size of 5-12 nm.

### EXAMPLE 4. Catalytic behaviour of the pre-catalyst A' in contact with the reaction gases at 2 MPa (Figure 4)

The catalytic behaviour of the catalyst is tested in the same reactor configuration as in Example 2. 260 mg (400-600 µm particle size) of pre-catalyst are diluted with 6300 mg SiC (600-800 µm particle size). The resulting mixture is brought into contact with 23.8 ml min⁻¹ CO₂, 71.3 ml min⁻¹ H₂ and 5 ml min⁻¹ N₂. The space velocity is 24000 h⁻¹, temperature is 180°C and pressure is 2 MPa. The reaction products undergo an online GC analysis (SCION-456-GC) using MS-13X and BR-Q Plot columns for TCD and FID detectors, respectively. Multiple analyses, each with a duration of 15 minutes, are acquired for a total of 1200 min of reaction time. **Figure 4** shows the time evolution in the conversion of CO₂ at 180°C, 2 MPa and 24000 h⁻¹. Selectivity to methane is 99.9%.

The data in **Figure 4** shows higher deactivation than what is observed in example 2.

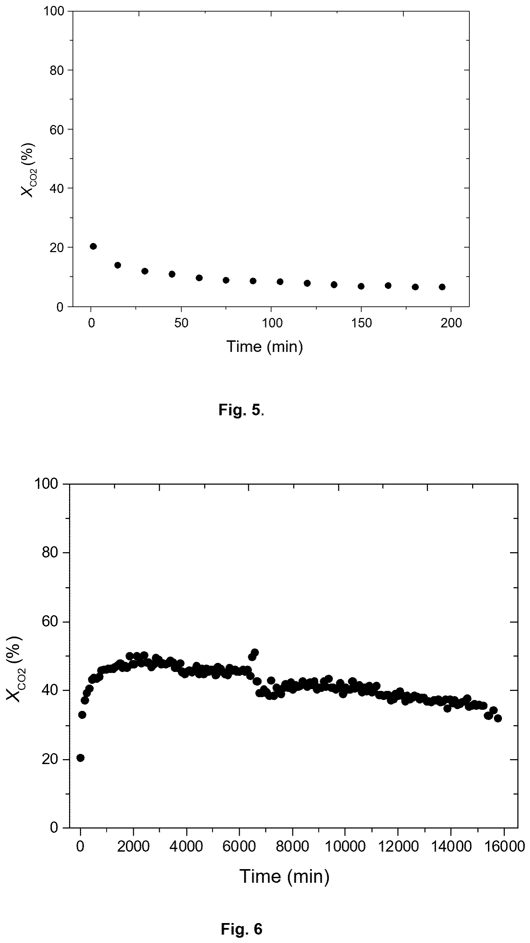

### EXAMPLE 5. Catalytic behaviour of the pre-catalyst A in contact with the reaction gases at 0.1 MPa (Figure 5)

The catalytic behaviour of the catalyst is tested in the same reactor configuration as in Example 2. 260 mg (400-600 µm particle size) of pre-catalyst are diluted with 6300 mg SiC (600-800 µm particle size). The resulting mixture is brought into contact with 23.8 ml min⁻¹ CO₂, 71.3 ml min⁻¹ H₂ and 5 ml min⁻¹ N₂. The space velocity is 24000 h⁻¹, temperature is 180°C and pressure is 0.1 MPa. Multiple analyses, each with a duration of 15 minutes, are acquired for a total of 200 min of reaction time. **Figure 5** shows the time evolution in the conversion of CO₂ at 180°C, 0.1 MPa and 24000 h⁻¹. Selectivity to methane is 99.9%.

The data in **Figure 5** shows a clear influence of pressure corresponding to a 7.5% conversion of CO₂ under steady-state conditions, and a methane yield of 17.55 mol_{CH4} L⁻¹ h⁻¹ (4.68 µmol s⁻¹ g_{cat}⁻¹).

### EXAMPLE 6. Pre-catalyst B

The pre-catalyst B is obtained by subjecting the pre-catalyst of RuO₂ (Aldrich) to hydrothermal conditions. Specifically, 120 mg glucose (Aldrich), 7 ml Milli-Q water and 100 mg RuO₂ (Aldrich) are mixed in ultrasound for 20 min. The resulting mixture is transferred to a 12.5 ml autoclave. The autoclave is placed in an oven at 175°C and kept under static conditions for 24 hours. After removal from the oven, the mixture is cooled to room temperature for 2 hours. The contents are filtered, washed with distilled water until no more foam is obtained and finally washed with acetone. The solid obtained is dried in an oven at 60°C for 12 hours.

### EXAMPLE 7. Catalytic behaviour of the pre-catalyst B in contact with the reaction gases at 2 MPa (Figure 6)

The catalytic behaviour of the catalyst is tested in the same reactor configuration as in Example 2. 260 mg (400-600 µm particle size) of pre-catalyst are diluted with 6300 mg SiC (600-800 µm particle size). The resulting mixture is brought into contact with 23.8 ml min⁻¹ CO₂, 71.3 ml min⁻¹ H₂ and 5 ml min⁻¹ N₂. The space velocity is 24000 h⁻¹, temperature is 180°C and pressure is 2 MPa. Multiple analyses, each with a duration of 15 minutes, are acquired for a total of 15769 min of reaction time. **Figure 6** shows the time evolution in the conversion of CO₂ at 180°C, 2 MPa and 24000 h⁻¹. Selectivity to methane is 99.9%.

The data in **Figure 6** corresponds to ~40% conversion of CO₂ under steady-state conditions, and a methane yield of 91.63 mol_{CH4} L⁻¹ h⁻¹ (24.50 µmol s⁻¹ g_{cat}⁻¹).

### EXAMPLE 8. Pre-catalyst C

1.12 g RuCl₃.xH₂O (Johnson Mattey, 40.7% wt Ru) are dissolved in 120 ml Milli-Q water. 8 ml octanoic acid are added drop by drop (Sigma Aldrich, >98%). The pH is adjusted to about 8 under stirring and by slowly adding a 1.5 M NaOH solution (Scharlau). Next, the mixture is heated to 80°C for 1 hour under continuous stirring. The supernatant is filtered under vacuum and washed first with water (until complete removal of chlorides and neutralisation of the mother liquor) and then three times with ethanol.

Lastly, the solid obtained is dried in an oven at 100°C for 3 hours and calcined in a muffle for 4 hours at 950°C (3°C/min). The X-ray diagram of the final solid corresponds to RuO₂ with a particle size of ~ 62.4 nm. The resulting material is used directly in a reaction and is referred to as pre-catalyst C.

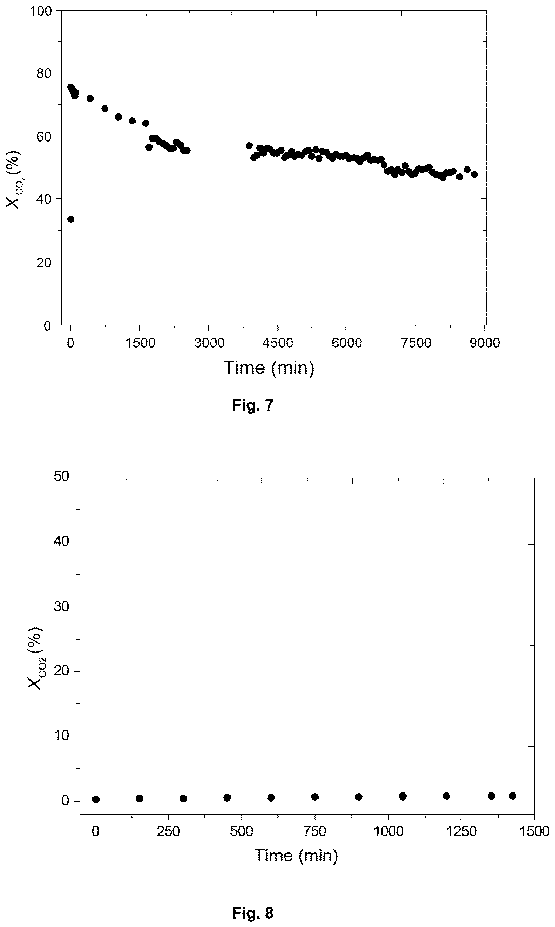

### EXAMPLE 9. Catalytic behaviour of the pre-catalyst C in contact with the reaction gases at 2 MPa (Figure 7)

The catalytic behaviour of the catalyst is tested in the same reactor configuration as in Example 2. 260 mg (400-600 µm particle size) of pre-catalyst are diluted with 6300 mg SiC (600-800 µm particle size). The resulting mixture is brought into contact with 23.8 ml min⁻¹ CO₂, 71.3 ml min⁻¹ H₂ and 5 ml min⁻¹ N₂. The space velocity is 24000 h⁻¹, temperature is 180°C and pressure is 2 MPa. Multiple analyses, each with a duration of 15 minutes, are acquired for a total of 9000 min of reaction time. Figure 7 shows the time evolution in the conversion of CO₂ of the catalyst at 180°C, 2 MPa and 24000 h⁻¹. Selectivity to methane is 99.9%.

The data in **Figure** 7 corresponds to ~50% conversion of CO₂ under steady-state conditions, and a methane yield of 117.04 mol_{CH4}·L⁻¹·h⁻¹ (31.21 µmol·s⁻¹·g_{cat}⁻¹).

### EXAMPLE 10 Catalyst D

The catalyst D is a Ru⁰ catalyst (Ruthenium Black) from Sigma Aldrich. The particle size according to the X-ray diffractogram is ~ 21 nm. The catalyst D is mixed by a physical method in a mortar with charcoal (Norit) in a proportion of 75:25 by weight.

### EXAMPLE 10. Catalytic behaviour of the reference catalyst D at 2 MPa (Figure 8)

The catalytic behaviour of the reference catalyst D is tested in the same reactor configuration as in Example 2. The catalyst is subjected to a pre-activation process in a stream of 20 vol% H₂, 80 vol% N₂, 100 ml min⁻¹ full flow at atmospheric pressure for 2 hours at 260°C. Subsequently, the reaction mixture is changed to 23.8 ml min⁻¹ CO₂, 71.3 ml min⁻¹ H₂ and 5 ml min⁻¹ N₂, at 100°C and 2 MPa. The space velocity is 24000 h⁻¹. Subsequently, the temperature is raised to 180°C (10°C min⁻¹). **Figure 8** shows the time evolution in the conversion of CO₂ at 180°C, 2 MPa and 24000 h⁻¹. Selectivity to methane is 99.9%.

The data in **Figure 8** corresponds to 0.54% CO₂ conversion, and a methane yield of 1.23 mol_{CH4}·L⁻¹·h⁻¹ (0.33 µmol·s⁻¹·g_{cat}⁻¹) which is significantly lower than the yield presented in example 2.

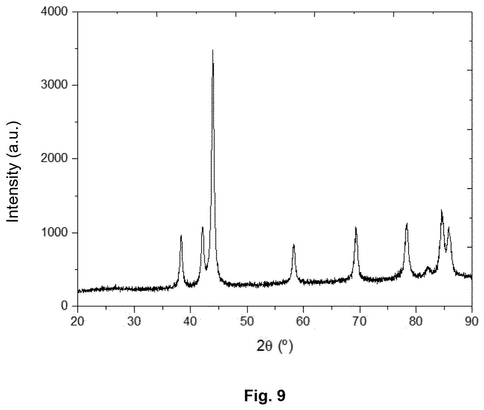

The X-ray diffraction of the catalyst D after being subjected to the reaction gas mixture is shown in **Figure 9****.** No peaks associated with the ruthenium oxo-carbonate phase are detected. The only peaks detected correspond to ruthenium metal.

## Claims

1. A ruthenium catalyst **characterised in that** it comprises, at least:
- Ru metal
- An organic or inorganic matrix
- Ru oxo-carbonate which acts as an active phase and has the following structure:
RuOₓC_{y}
Where x is between 1 and 3.
Where y is between 0.3 and 0.8

2. The catalyst according to claim 1, **characterised in that** the active phase further comprises nitrogen in an amount between 0.1-0.5 molar ratio.

3. The catalyst according to any of claims 1 and 2, **characterised in that** the oxo-carbonate phase crystallises in a monoclinic phase containing carbon in interstitial positions.

4. The catalyst according to any of the preceding claims, **characterised in that** the organic or inorganic matrix is selected from silica, alumina, metal oxides, zeolites, carbon and combinations thereof.

5. The catalyst according to claim 4, **characterised in that** the organic matrix is carbon.

6. The catalyst according to any of the preceding claims, **characterised in that** the catalyst further comprises a metal selected from Pd, Co, Ni, In, alkalis, alkaline earth metals and combinations thereof.

7. A method for obtaining a catalyst according to one of the preceding claims, **characterised in that** it comprises, at least, putting a ruthenium pre-catalyst in contact with a reaction gas mixture comprising, at least, H₂, N₂ and CO₂.

8. The method for obtaining a catalyst according to claim 7, **characterised in that** the pre-catalyst is selected from RuO₂, a mixture of RuO₂ with an organic or inorganic matrix and a mixture of RuO₂ with other metal oxides selected from PdO₂, Co₃O₄, NiO, In₂O₃, alkalis, alkaline earth metals and combinations thereof.

9. The method for obtaining a catalyst according to any of claims 7 and 8, **characterised in that** the pre-catalyst is a mixture of RuO₂ with an organic or inorganic matrix in a proportion by weight between 99.9 and 10 RuO₂: and 0.1 and 90 organic or inorganic matrix.

10. The method for obtaining a catalyst according to any of the preceding claims, **characterised in that** the H₂/CO₂ molar ratio in the reaction gas mixture is between 1 and 6.

11. Use of the catalyst described in claims 1 to 6 and obtained according to the method described in claims 7 to 10 for obtaining methane by hydrogenation of CO₂ at a temperature between 50 and 200°C, and a space velocity (GHSV) between 10714 and 42857 h⁻¹, in the amount of hydrogen (H₂/CO₂ < 6) and a pressure between atmospheric and 5 MPa.

12. The use of the catalyst according to claim 11, **characterised in that** the hydrogenation reaction of CO₂ is carried out in a fixed bed reactor, fluidised bed, slurry reactor or in a membrane reactor.
